# FASCICULE DE BREVET EUROPEEN

(11) **EP 2 807 141 B1**
(45) Date de publication et mention de la délivrance du brevet: **21.10.2015**
(21) Numéro de dépôt: 13705206.4
(22) Date de dépôt: 14.01.2013
(51) Int. Cl.: C07C 67/03, C07C 67/54, C07C 69/54

(54) **PROCEDE DE PRODUCTION D'ACRYLATE DE 2-OCTYLE PAR TRANSESTERIFICATION**
VERFAHREN ZUR HERSTELLUNG VON 2-OCTYL-ACRYLAT DURCH UMESTERUNG
METHOD FOR THE PRODUCTION OF 2-OCTYL ACRYLATE BY MEANS OF TRANSESTERIFICATION

(30) Priorité: 23.01.2012 FR 1250606
(43) Date de publication de la demande: 03.12.2014
(73) Titulaire: Arkema France, 92700 Colombes (FR)
(72) Inventeur: RIONDEL, Alain, F-77178 Saint Pathus (FR); GRAIRE, Coralie, F-69290 Grezieu-la-Varenne (FR); ESCH, Marc, 57450 Theding (FR)
(74) Mandataire: Bonnel, Claudine
(86) Numéro de dépôt international: PCT/FR2013/050079
(87) Numéro de publication internationale: WO 2013/110877

(56) Documents cités:
- US-B2- 6 977 310
- US-B2- 7 268 251
- DANNI LIU ET AL: "Rational Design of Pseudozyma antarctica Lipase B Yielding a General Esterification Catalyst", CHEMBIOCHEM, vol. 11, no. 6, 12 avril 2010 (2010-04-12) , pages 789-795, XP055033062, ISSN: 1439-4227, DOI: 10.1002/cbic.200900776

## Description

### DOMAINE DE L'INVENTION

La présente invention concerne la production d'acrylate de 2-octyle selon un procédé en continu par transestérafication.

### ARRIERE-PLAN TECHNIQUE

Il est connu de produire des esters acryliques en mettant en oeuvre une réaction de transestérification entre un acrylate d'alcool léger (dénommé acrylate léger) et un alcool lourd

Cette réaction est une réaction catalysée équilibrée avec génération d'alcool léger, selon la formule (I).

CH₂=CH-COOR₁ + R₂-OH ⇔ CH₂=CH-COOR₂ + R₁-OH

Il est nécessaire d'éliminer l'alcool léger produit au cours de la réaction pour déplacer l'équilibre dans le sens de la production de l'ester acrylique.

Cette réaction s'accompagne généralement de réactions secondaires produisant des impuretés qu'il est nécessaire d'éliminer en vue d'obtenir l'ester acrylique avec une pureté élevée satisfaisant aux exigences techniques liées à son utilisation finale en tant que monomère pour fabriquer des polymères utilisables dans de nombreux domaines d'application.

Par ailleurs, pour des raisons économiques évidentes, les produits valorisables présents dans le mélange brut réactionnel, notamment les réactifs non réagis et le catalyseur, sont dans la mesure du possible recyclés au sein du procédé.

A ces fins, on procède généralement à un processus de séparation/purification comportant un ensemble de distillations, d'extractions, et/ou décantations, qui est à la fois relativement complexe à mettre en oeuvre, notamment du fait de la présence de mélanges azéotropiques, et coûteux sur le plan énergétique.

Différents procédés de transestérification pour produire des esters acryliques ont déjà été décrits dans l'art antérieur.

On peut citer par exemple le document US 7,268,251 dans lequel l'effluent réactionnel de la transestérification est traité de la façon suivante :
soit on sépare tout d'abord la majeure partie de l'ester acrylique recherché et on l'isole ensuite du catalyseur utilisé par distillation (séparation de catalyseur),
soit on l'isole tout d'abord du catalyseur utilisé par distillation (séparation de catalyseur) et ensuite on sépare la majeure partie de l'ester acrylique,
et ensuite on sépare par distillation du mélange obtenu, les composés à point d'ébullition inférieur à celui de l'ester acrylique recherché (séparation des substances à bas point d'ébullition) et ensuite on distille l'ester acrylique (distillation à l'état pur).

Ce procédé nécessite la mise en oeuvre d'au moins quatre colonnes de distillation ou de rectification, dont un évaporateur pour séparer le catalyseur, généralement un alcoolate de titane.

Même si le procédé décrit dans le document US 7,268,251 concerne la fabrication d' acrylates d'alkyle par transestérification à partir d'un acrylate d'alkyle et d'un alcool présentant une longueur de chaine supérieure d'au moins un carbone par rapport à la chaine alkyle de l'acrylate de départ, ce procédé n'est illustré qu'avec la fabrication d'acrylate de diméthylaminoéthyle à partir de diméthylaminoéthanol et d'acrylate de méthyle ou d'acrylate d'éthyle dans une cascade de deux réacteurs.

Il s'avère que le procédé décrit dans le document US 7,268,251 est compliqué à mettre en oeuvre à l'échelle industrielle, du fait de l'optimisation des conditions opératoires de la succession des quatre éléments de distillation/rectification, pour obtenir un produit d'une grande pureté et une productivité satisfaisante.

Le document US 6,977,310 décrit un procédé de fabrication en continu d'esters alkyliques d'acide (méth)acrylique à partir de (méth)acrylate de méthyle et d'un alcool en C₂-C₁₂, en présence d'un titanate de tétraalkyle comme catalyseur de transestérification. Ce procédé consiste à soumettre le mélange réactionnel à une distillation sous pression réduite séparant les composés aisément volatils (réactifs non réagis) ; puis, la fraction résultante sortant en pied de colonne, comprenant l'ester produit, le catalyseur, les inhibiteurs de polymérisation et les produits secondaires à point d'ébullition élevé, est envoyée vers un étage de distillation sous vide qui permet de récupérer en tête l'ester produit de grande pureté Cet étage de distillation sous vide comprend notamment un évaporateur à film, combiné avec une colonne de distillation pour une élimination complète des produits à point d'ébullition élevé dans l'ester produit.

Ce procédé est illustré avec la fabrication de méthacrylate de butyle et de méthacrylate d'isobutyle, respectivement à partir de butanol et d'isobutanol.

Le procédé décrit dans le document US 6,977,310 met en oeuvre un évaporateur à film pour éviter toute dégradation du catalyseur et toute formation d'éthers du réactif alcool Ce procédé ne prévoit pas en outre le recyclage du catalyseur.

De façon surprenante, il a été constaté que dans le cas de l'acrylate de 2-octyle, l'élimination du catalyseur à l'aide d'un bouilleur classique et non d'un évaporateur à film, ne conduit pas à la formation d'impuretés comme les éthers ou les octènes.

La Société Déposante cherchant à résoudre les différents problèmes des procédés précités a ainsi découvert un procédé de fabrication simplifié pour produire de l'acrylate de 2-octyle de très haute pureté avec un rendement élevé, tout en incluant le recyclage des produits valorisables tels que les réactifs non réagis et le catalyseur, et présentant ainsi une productivité compatible avec une fabrication à l'échelle industrielle.

La solution proposée consiste à utiliser du titanate d'éthyle en solution dans le 2-octanol ou du titanate de 2-octyle comme catalyseur de transestérification, et à mettre en oeuvre un train de purification ne comportant que deux colonnes de distillation.

La présente invention permet en outre de produire un ester acrylique comportant du carbone d'origine renouvelable lié à l'utilisation du 2-octanol, qui est un alcool dérivé de matières végétales.

### RESUME DE L'INVENTION

La présente invention a pour objet un procédé de production en continu d'acrylate de 2-octyle par réaction de transestérification entre un acrylate d'alcool léger et du 2-octanol en présence d'un titanate d'alkyle comme catalyseur de transesténfication et d'au moins un inhibiteur de polymérisation, le mélange azéotropique composé d'acrylate d'alcool léger et d'alcool léger généré par la réaction de transestérification étant soutiré en continu pendant la réaction, le mélange réactionnel étant soumis à un traitement de purification comportant deux colonnes de distillation, afin d'obtenir d'une part l'acrylate de 2-octyle pur, d'autre part les composés 2-octanol et acrylate d'alcool léger non réagis destinés à être recyclés, et aussi le catalyseur destiné à être recyclé, procédé caractérisé en ce que :
- on choisit le catalyseur parmi le titanate d'éthyle en solution dans le 2-octanol et le titanate de 2-octyle ;
- on adresse à une première colonne de distillation (B) sous pression réduite, le mélange brut réactionnel comprenant l'acrylate de 2-octyle recherché avec, comme produits légers, le 2-octanol et l'acrylate d'alcool léger n'ayant pas réagi, et comme produits lourds le catalyseur, le ou les inhibiteurs de polymérisation ainsi que des produits de réaction lourds, et on effectue, dans ladite première colonne (B), une distillation permettant d'obtenir :
   o en tête, un flux composé essentiellement de 2-octanol et d'acrylate d'alcool léger n'ayant pas réagi, avec une fraction minoritaire d'acrylate de 2-octyle, et
   o en pied, un flux comprenant l'acrylate de 2-octyle, le catalyseur, le ou les inhibiteurs de polymérisation ainsi que les produits de réaction lourds, et des traces de composés légers ; puis
- on adresse le flux de pied de la première colonne de distillation (B) à une seconde colonne de distillation (C) sous pression réduite, dans laquelle est effectuée une distillation permettant d'obtenir :
   o en tête, l'acrylate de 2-octyle pur recherché ; et
   o en pied, le catalyseur, le ou les inhibiteurs de polymérisation ainsi que les produits de réaction lourds, et de l'acrylate de 2-octyle.
- on recycle à la réaction, au moins en partie le flux de pied de la seconde colonne de distillation (C).

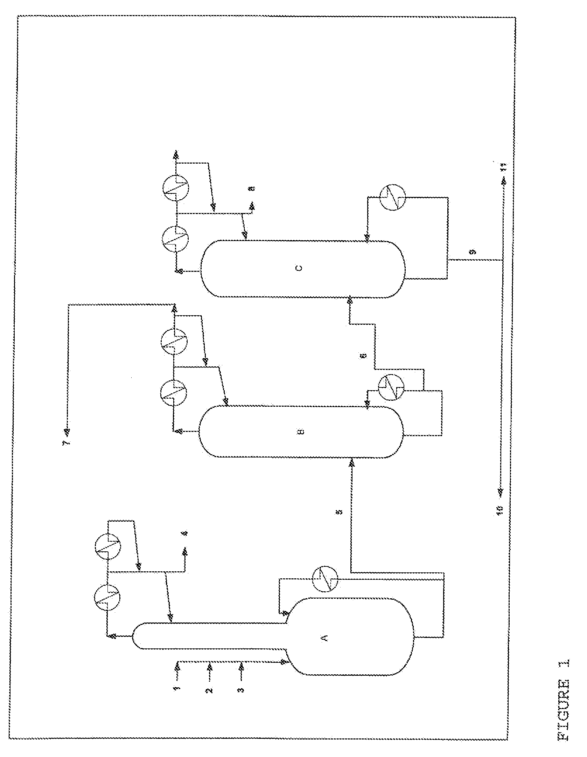

L'invention est maintenant décrite plus en détail et de façon non limitative dans la description qui suit, en référence à la figure unique annexée qui représente de manière schématique une installation permettant de mettre en oeuvre le procédé selon l'invention.

### DESCRIPTION DETAILLEE

L'un des objectifs de l'invention est d'utiliser des matières premières d'origine naturelle et renouvelable, c'est-à-dire bio-sourcées.

Le 2-octanol utilisé dans le procédé selon l'invention est un alcool d'origine renouvelable, en particulier il peut être obtenu par traitement alcalin de l'acide ricinoléique dérivé de l'huile de ricin.

L'acrylate d'alcool léger mis en oeuvre comme matière première dans le procédé selon l'invention est obtenu par estérification directe de l'acide acrylique essentiellement produit sur le plan industriel à partir de propylène, avec un alcool léger, généralement le méthanol ou l'éthanol.

Indépendamment de la mise en oeuvre du 2-octanol d'origine renouvelable, l'invention s'étend à l'utilisation d'un acrylate d'alcool léger dérivé d'acide acrylique d'origine renouvelable, pouvant être en particulier obtenu à partir de glycérol, selon un procédé comportant une première étape de déshydratation du glycérol en acroléine suivie d'une étape d'oxydation en phase gaz de l'acroléine ainsi obtenue, ou obtenu par déshydratation des acides 2-hydroxypropionique (acide lactique) ou 3-hydroxypropionique et de leurs esters.

L'invention s'étend également à l'utilisation d'un acrylate d'alcool léger dérivé d'un alcool biossourcé, tel que le bioéthanol.

D'une manière générale, la réaction de transestérification est réalisée dans un réacteur agité (A), chauffé par un échangeur externe et surmonté d'une colonne à distiller, avec un rapport molaire acrylate d'alcool léger / 2-octanol pouvant aller de 1 à 3, de préférence compris entre 1,3 et 1.8.

Comme acrylate d'alcool léger, on utilise l'acrylate de méthyle, l'acrylate d'éthyle ou l'acrylate de butyle, de préférence l'acrylate d'éthyle.

Le catalyseur de transestérification est le titanate d'éthyle en solution dans le 2-octanol, par exemple une solution à 90% de titanate d'éthyle dans le 2-octanol, ou le titanate de 2-octyle, obtenu préalablement par réaction à 100°C du titanate d'éthyle avec le 2-octanol), de préférence le titanate de 2-octyle.

On utilise le catalyseur à raison de 5.10⁻⁴ à 5.10⁻² mole par mole de 2-octanol, de préférence à raison de 10⁻³ à 10⁻² mole par mole de 2-octanol

On conduit généralement la réaction de transestérification dans le réacteur (A) à une pression comprise entre 500 mm Hg (0,67 10⁵ Pa) et la pression atmosphérique, et à une température allant de 90°C à 130°C, de préférence de 100°C à 120°C.

La réaction est effectuée en présence d'un ou plusieurs inhibiteurs de polymérisation qui sont introduits dans le réacteur, à raison de 1000 à 5000 ppm par rapport au mélange brut réactionnel Comme inhibiteurs de polymérisation utilisables, on peut citer par exemple la phénothiazine, l'hydroquinone, l'éther monométhylique d'hydroquinone, le diterbutyl para-crésol (BHT), le TEMPO (2,2,6,6-tétraméthyl-1-pipendinyloxy), le di-tertiobutylcatéchol, ou les dérivés du TEMPO, tel que le 4-hydroxy TEMPO (4-OHTEMPO), seuls ou leurs mélanges en toutes proportions. Un ajout supplémentaire d'inhibiteur de polymérisation est généralement effectué au niveau du traitement ultérieur de purification, en particulier au niveau de chacune des colonnes de distillation.

L'alcool léger formé par la réaction de transestérification est entraîné en continu par distillation dans la colonne surmontant le réacteur sous forme d'un mélange azéotropique avec l'acrylate d'alcool léger. Ce mélange est avantageusement recyclé à l'unité de synthèse de l'acrylate léger.

Après réaction avec un temps de séjour dans le réacteur généralement compris entre 3 et 6 heures, le mélange brut réactionnel (5) contient l'acrylate de 2-octyle recherché avec, comme produits légers, le 2-octanol et l'acrylate d'alcool léger n'ayant pas réagi, et comme produits lourds, le catalyseur, le ou les inhibiteurs de polymérisation ainsi que des sous-produits de réaction lourds.

Le mélange réactionnel est soumis à un traitement de purification comportant deux colonnes de distillation (B) et (C), afin d'obtenir d'une part l'acrylate de 2-octyle pur, d'autre part les composés 2-octanol et acrylate d'alcool léger non réagis destinés à être recyclés, et aussi le catalyseur destiné à être recyclé

La première colonne de distillation (B) fonctionne généralement sous une pression allant 20 à 50 mm Hg (0,027 10⁵ Pa à 0.067 10⁵ Pa) à une température de pied allant de 120°C à 150°C.

Le flux (7) de tête de colonne (B) est constitué principalement des produits légers non réagis (acrylate d'alcool léger et 2-octanol), avec une fraction minoritaire d'acrylate de 2-octyle produit Ce flux (7) peut être avantageusement recyclé à la réaction de transestérification dans le réacteur (A).

Le flux (6) de pied de colonne (B) est constitué principalement d'acrylate de 2-octyle avec le catalyseur, les inhibiteurs de polymérisation et les sous-produits lourds et peut contenir des traces résiduelles de composés légers.

Ce flux (6) est soumis à une distillation dans une seconde colonne (C) qui fonctionne généralement sous une pression de 20 à 50 mm Hg (0,027 10⁵ Pa à 0,067 10⁵ Pa) et une température allant de 120°C à 150°C

La colonne (C) permet de récupérer en tête (8), l'acrylate de 2-octyle purifié.

En pied de la colonne (C), on sépare dans un flux (9), le catalyseur, les sous-produits lourds, les inhibiteurs de polymérisation, et de l'acrylate de 2-octyle.

Une partie (10) de ce flux (9) est avantageusement recyclée au niveau de la réaction dans le réacteur (A), le restant (flux 11) étant envoyé en destruction.

L'acrylate de 2-octyle pur (8), récupéré en tête de la colonne (C), présente une pureté supérieure à 99.3 %, voire supérieure ou égale à 99,6%.

Les exemples suivants illustrent la présente invention sans toutefois en limiter la portée.

### PARTIE EXPERIMENTALE

Dans les exemples, les pourcentages sont indiqués en poids sauf indication contraire et les abréviations suivantes ont été utilisées :
AE : acrylate d'éthyle
A2OCT : acrylate de 2-octyle
PTZ. phénothiazine

### Exemple 1 (selon l'invention)

Dans un réacteur A parfaitement agité, chauffé par un échangeur externe et surmonté d'une colonne à distiller à garnissage de 12 plateaux théoriques, on charge un mélange comprenant les réactifs acrylate d'éthyle et 2-octanol, du titanate d'éthyle en solution à 90% dans le 2-octanol comme catalyseur avec de la PTZ comme inhibiteur, dans les proportions massiques 53,8/45,6/0,6.

On met le réacteur en chauffe sous bullage d'air et dès que la température atteint 115°C sous 500 mm Hg (0,67 10⁵ Pa), on introduit en continu l'AE (3) stabilisé avec 2500 ppm de PTZ. le 2-octanol (1) et un mélange (2) de titanate d'éthyle en solution dans 2-octanol (mélange à 90 %) dans des proportions massiques 53,8/45,6/0,6.

En tête de colonne, on soutire en continu l'azéotrope AE/Ethanol (4) avec une composition massique 35/65. Ce mélange (4) est recyclé directement sur l'atelier de fabrication de l'AE.

Le brut réactionnel (5) contient l'A2OCT formé, l'AE non réagi, le 2-octanol non-réagi et un mélange comprenant le catalyseur avec les inhibiteurs de polymérisation et des dérivés lourds, dans des proportions massiques 73/20,1/6,3/0,6. Le brut réactionnel (5) est envoyé en continu vers une première colonne de distillation B de 15 plateaux théoriques opérant sous pression réduite et chauffée par un échangeur externe. En tête de colonne B, on introduit un mélange à 2500 ppm de PTZ dans l'AE.

La colonne B sépare en tête un mélange (7) comprenant les réactifs non réagis, AE et 2-Octanol, avec une fraction minoritaire de A2OCT, de composition massique 67/21/13 qui est renvoyé à l'étape de réaction.

En pied de la colonne B, on récupère un mélange (6) enrichi en A20CT et comprenant les inhibiteurs de polymérisation, le catalyseur et des dérivés lourds : ce mélange a la composition massique :
- A2OCT 97.8%
- AE : 100 ppm
- 2-octanol : 600 ppm
- dérivés lourds + inhibiteurs + catalyseur : 2.1%

Ce mélange (6) est envoyé vers une seconde colonne de distillation C. En tête de colonne C, on introduit un mélange à 2500 ppm d'EMHQ dans l'A2OCT. La colonne C sépare en tête l'A2OCT (8) purifié, et en pied un flux (9) contenant majoritairement le catalyseur, les dérivés lourds, les inhibiteurs de polymérisation et de l'acrylate de 2-octyle. Ce flux (9) est recyclé en grande partie (de l'ordre de 90% en poids) vers le réacteur A (flux 10), la partie restante (11) étant envoyée en destruction.

L'acrylate de 2-octyle a la pureté suivante :
A2OCT : 99.5%
AE : 500 ppm
2-Octanol : 1500 ppm

### Exemple 2 (comparatif)

On a réalisé la même synthèse qu'à l'exemple 1, mais en utilisant comme catalyseur le titanate de butyle en remplacement du titanate d'éthyle.

Dans ce cas, le flux (7) distillé en tête de la colonne B, contient outre les réactifs non réagis avec une fraction minoritaire de A2OCT, 15% d'acrylate de butyle provenant de la réaction du catalyseur avec l'AE.

Ce flux (7), destiné à être recyclé à l'étape réactionnelle, a nécessité une purification préalable par distillation sur une colonne supplémentaire pour éliminer l'acrylate de butyle, afin de limiter l'accumulation au cours du temps d'acrylate de butyle dans l'installation, et le risque de pollution de l'A2OCT purifié

### Exemple 3 (comparatif)

On a réalisé la même synthèse qu'à l'exemple 1, mais en utilisant comme catalyseur le titanate de 2-éthylhexyle en remplacement du titanate d'éthyle.

Dans ce cas, on a obtenu en tête de la colonne C, de l'A2OCT (9) de pureté 97,3% en raison de la présence de 2% d'acrylate de 2-éthylhexyle dans le produit purifié. L'acrylate de 2-octyle ainsi obtenu n'offre pas les mêmes performances dans les adhésifs sensibles à la pression qu'un A2OCT de pureté 99,5%.

## Revendications

1. Procédé de production en continu d'acrylate de 2-octyle par réaction de transestérification entre un acrylate d'alcool léger et du 2-octanol en présence d'un titanate d'alkyle comme catalyseur de transestérification et d'au moins un inhibiteur de polymérisation, le mélange azéotropique composé d'acrylate d'alcool léger et d'alcool léger généré par la réaction de transestérification étant soutiré en continu pendant la réaction, le mélange réactionnel étant soumis à un traitement de purification comportant deux colonnes de distillation, afin d'obtenir d'une part l'acrylate de 2-octyle pur, d'autre part les composés 2-octanol et acrylate d'alcool léger non réagis destinés à être recyclés, et aussi le catalyseur destiné à être recyclé, procédé **caractérisé en ce que** :
- on choisit le catalyseur parmi le titanate d'éthyle en solution dans le 2-octanol et le titanate de 2-octyle ;
- on adresse à une première colonne de distillation (B) sous pression réduite, le mélange brut réactionnel comprenant l'acrylate de 2-octyle recherché avec, comme produits légers, le 2-octanol et l'acrylate d'alcool léger n'ayant pas réagi, et comme produits lourds, le catalyseur, le ou les inhibiteurs de polymérisation ainsi que des produits de réaction lourds, et on effectue, dans ladite première colonne (B), une distillation permettant d'obtenir :
∘ en tête, un flux composé essentiellement de 2-octanol et d'acrylate d'alcool léger n'ayant pas réagi, avec une fraction minoritaire d'acrylate de 2-octyle, et
o en pied, un flux comprenant l'acrylate de 2-octyle, le catalyseur, le ou les inhibiteurs de polymérisation ainsi que les produits de réaction lourds, et des traces de composés légers ; puis
- on adresse le flux de pied de la première colonne de distillation (B) à une seconde colonne de distillation (C) sous pression réduite, dans laquelle est effectuée une distillation permettant d'obtenir :
o en tête, l'acrylate de 2-octyle pur recherché ; et
o en pied, le catalyseur, le ou les inhibiteurs de polymérisation ainsi que les produits de réaction lourds, et de l'acrylate de 2-octyle.
- on recycle à la réaction, au moins en partie le flux de pied de la seconde colonne de distillation (C).

2. Procédé selon la revendication 1 **caractérisé en ce que** le catalyseur est le titanate de 2-octyle.

3. Procédé selon la revendication 1 ou 2 **caractérisé en ce que** l'on utilise le catalyseur à raison de 5.10⁻⁴ à 5.10⁻² mole par mole de 2-octanol, de préférence à raison de 10⁻³ à 10⁻² mole par mole de 2-octanol.

4. Procédé selon l'une quelconque des revendications précédentes **caractérisé en ce que** la réaction est réalisée à partir d'acrylate d'éthyle.

5. Procédé selon l'une quelconque des revendications précédentes **caractérisé en ce que** le rapport molaire acrylate d'alcool léger / 2-octanol va de 1 à 3, de préférence compris entre 1,3 et 1,8.

6. Procédé selon l'une quelconque des revendications précédentes **caractérisé en ce que** la réaction de transestérification est effectuée à une pression comprise entre 500 mm Hg (0,67 10⁵ Pa) et la pression atmosphérique (10⁵ Pa), et à une température allant de 90°C à 130°C, de préférence de 100°C à 120°C.

7. Procédé selon l'une quelconque des revendications précédentes **caractérisé en ce que** l'acrylate d'alcool léger est l'acrylate d'éthyle d'origine renouvelable.

## Patentansprüche

1. Verfahren zur kontinuierlichen Herstellung von 2-Octylacrylat durch Umesterungsreaktion zwischen einem Acrylat eines leichten Alkohols und 2-Octanol in Gegenwart eines Alkyltitanats als Umesterungskatalysator und mindestens eines Polymerisationsinhibitors, wobei die azeotrope Mischung, die aus Acrylat eines leichten Alkohols und durch die Umesterungsreaktion gebildetem leichtem Alkohol besteht, während der Reaktion kontinuierlich abgezogen wird, wobei die Reaktionsmischung einer Reinigungsbehandlung mit zwei Destillationssäulen unterworfen wird, wobei man einerseits reines 2-Octylacrylat, andererseits die zur Rezyklierung bestimmten nicht umgesetzten Verbindungen 2-Octanol und Acrylat eines leichten Alkohols sowie den zur Rezyklierung bestimmten Katalysator erhält, wobei das Verfahren **dadurch gekennzeichnet ist, dass**:
- der Katalysator aus Ethyltitanat in Lösung in 2-Octanol und 2-Octyltitanat ausgewählt wird;
- die rohe Reaktionsmischung, die das gewünschte 2-Octylacrylat mit dem nicht umgesetzten 2-Octanol und Acrylat eines leichten Alkohols als leichten Produkten und dem Katalysator, dem Polymerisationsinhibitor bzw. den Polymerisationsinhibitoren sowie schweren Reaktionsprodukten als schweren Produkten umfasst, einer ersten Destillationssäule (B) unter vermindertem Druck zugeführt wird und in der ersten Säule (B) eine Destillation durchgeführt wird, die es ermöglicht, Folgendes zu erhalten:
∘ am Kopf einen Strom, der im Wesentlichen aus nicht umgesetztem 2-Octanol und Acrylat eines leichten Alkohols mit einer untergeordneten Fraktion von 2-Octylacrylat besteht, und
∘ am Sumpf einen Strom, der das 2-Octylacrylat, den Katalysator, den Polymerisationsinhibitor bzw. die Polymerisationsinhibitoren sowie die schweren Reaktionsprodukte und Spuren von leichten Verbindungen umfasst; dann
- der Sumpfstrom aus der ersten Destillationssäule (B) einer zweiten Destillationssäule (C) unter vermindertem Druck zugeführt wird, in der eine Destillation durchgeführt wird, die es ermöglicht, Folgendes zu erhalten:
∘ am Kopf das gewünschte reine 2-Octylacrylat; und
o am Sumpf den Katalysator, den Polymerisationsinhibitor bzw. die Polymerisationsinhibitoren sowie die schweren Reaktionsprodukte und 2-Octylacrylat;
- der Sumpfstrom aus der zweiten Destillationssäule (C) zumindest teilweise zur Reaktion rezykliert wird.

2. Verfahren nach Anspruch 1, **dadurch gekennzeichnet, dass** es sich bei dem Katalysator um 2-Octyltitanat handelt.

3. Verfahren nach Anspruch 1 oder 2, **dadurch gekennzeichnet, dass** man den Katalysator in einer Menge von 5·10⁻⁴ bis 5·10⁻² mol pro Mol 2-Octanol und vorzugsweise in einer Menge von 10⁻³ bis 10⁻² mol pro Mol 2-Octanol verwendet.

4. Verfahren nach einem der vorhergehenden Ansprüche, **dadurch gekennzeichnet, dass** die Reaktion ausgehend von Ethylacrylat durchgeführt wird.

5. Verfahren nach einem der vorhergehenden Ansprüche, **dadurch gekennzeichnet, dass** das Molverhältnis von Acrylat eines leichten Alkohols zu 2-Octanol im Bereich von 1 bis 3 und vorzugsweise zwischen 1,3 und 1,8 liegt.

6. Verfahren nach einem der vorhergehenden Ansprüche, **dadurch gekennzeichnet, dass** die Umesterungsreaktion bei einem Druck zwischen 500 mm Hg (0,67·10⁵ Pa) und Normaldruck (10⁵ Pa) und einer Temperatur im Bereich von 90°C bis 130°C und vorzugsweise von 100°C bis 120°C durchgeführt wird.

7. Verfahren nach einem der vorhergehenden Ansprüche, **dadurch gekennzeichnet, dass** es sich bei dem Acrylat eines leichten Alkohols um Ethylacrylat erneuerbaren Ursprungs handelt.

## Claims

1. A process for the continuous production of 2-octyl acrylate by a transesterification reaction between a light alcohol acrylate and 2-octanol in the presence of an alkyl titanate as transesterification catalyst and at least one polymerization inhibitor, the azeotropic mixture composed of light alcohol acrylate and of light alcohol generated by the transesterification reaction being withdrawn continuously during the reaction, the reaction mixture being subjected to a purification treatment comprising two distillation columns, in order to obtain, on the one hand, the pure 2-octyl acrylate and, on the other hand, the unreacted 2-octanol and light alcohol acrylate compounds intended to be recycled, and also the catalyst intended to be recycled, which process is **characterized in that**:
- the catalyst is chosen from ethyl titanate in solution in 2-octanol and 2-octyl titanate;
- the crude reaction mixture comprising the desired 2-octyl acrylate with, as light products, the unreacted 2-octanol and light alcohol acrylate and, as heavy products, the catalyst, the polymerization inhibitor or inhibitors and also heavy reaction products is sent to a first distillation column (B) under reduced pressure and a distillation is carried out, in said first column (B), which makes it possible to obtain:
o at the top, a stream composed essentially of unreacted 2-octanol and light alcohol acrylate, with a minor fraction of 2-octyl acrylate, and
o at the bottom, a stream comprising 2-octyl acrylate, the catalyst, the polymerization inhibitor or inhibitors and the heavy reaction products, and traces of light compounds; then
- the bottom stream from the first distillation column (B) is sent to a second distillation column (C) under reduced pressure, in which a distillation is carried out which makes it possible to obtain:
o at the top, the desired pure 2-octyl acrylate; and
o at the bottom, the catalyst, the polymerization inhibitor or inhibitors and the heavy reaction products, and 2-octyl acrylate;
- the bottom stream from the second distillation column (C) is recycled to the reaction, at least in part.

2. The process as claimed in claim 1, **characterized in that** the catalyst is 2-octyl titanate.

3. The process as claimed in claim 1 or claim 2, **characterized in that** the catalyst is used in a proportion of 5 × 10⁻⁴ to 5 × 10⁻² mol per mole of 2-octanol, preferably in a proportion of 10⁻³ to 10⁻² mol per mole of 2-octanol.

4. The process as claimed in any one of the preceding claims, **characterized in that** the reaction is carried out starting from ethyl acrylate.

5. The process as claimed in any one of the preceding claims, **characterized in that** the light alcohol acrylate/2-octanol molar ratio ranges from 1 to 3, preferably between 1.3 and 1.8.

6. The process as claimed in any one of the preceding claims, **characterized in that** the transesterification reaction is carried out at a pressure of between 500 mmHg (0.67 × 10⁵ Pa) and atmospheric pressure (10⁵ Pa) and at a temperature ranging from 90°C to 130°C, preferably from 100°C to 120°C.

7. The process as claimed in any one of the preceding claims, **characterized in that** the light alcohol acrylate is ethyl acrylate of renewable origin.
